# EUROPEAN PATENT SPECIFICATION

(11) **EP 3 760 213 B1**
(45) Date of publication and mention of the grant of the patent: **27.03.2024**
(21) Application number: 19761185.8
(22) Date of filing: 27.02.2019
(51) Int. Cl.: A61K 35/768, A61K 35/761, A61K 35/763, A61K 31/17, A61P 35/00, C12N 7/00

(54) **PHARMACEUTICAL COMPOSITION FOR PREVENTING OR TREATING CANCER COMPRISING ANTICANCER VIRUS AND HYDROXYUREA AS EFFECTIVE COMPONENTS**
PHARMAZEUTISCHE ZUSAMMENSETZUNG ZUR VORBEUGUNG ODER BEHANDLUNG VON KREBS MIT EINEM ANTIKREBSVIRUS UND HYDROXYHARNSTOFF ALS WIRKSAME BESTANDTEILE
COMPOSITION PHARMACEUTIQUE POUR PRÉVENIR OU TRAITER LE CANCER, COMPRENANT COMME PRINCIPES ACTIFS UN VIRUS ANTICANCÉREUX ET DE L'HYDROXYURÉE

(30) Priority: 28.02.2018 KR 20180024461
(43) Date of publication of application: 06.01.2021
(60) Divisional application: 24155126.6
(73) Proprietor: Bionoxx Inc., Seongnam-si, Gyeonggi-do 13554 (KR)
(72) Inventor: HWANG, Tae-Ho, Yangsan-si, Gyeongsangnam-do 50651 (KR); CHO, Mong, Yangsan-si, Gyeongsangnam-do 50658 (KR)
(74) Representative: Dehns
(86) International application number: PCT/KR2019/002376
(87) International publication number: WO 2019/168346

(56) References cited:
- WO-A1-2017/205674
- US-A1- 2009 317 456
- US-A1- 2014 234 257
- C. J. BREITBACH ET AL: "Oncolytic Vaccinia Virus Disrupts Tumor-Associated Vasculature in Humans", CANCER RESEARCH, vol. 73, no. 4, 7 February 2013 (2013-02-07), pages 1265-1275, XP055299193, US ISSN: 0008-5472, DOI: 10.1158/0008-5472.CAN-12-2687
- BOUCHER, P.D.: "Synergistic enhancement of herpes simplex virus thymidine kinase/ganciclovir-mediated cytotoxicity by hydroxyurea", Cancer Research, vol. 60, no. 6, 2000, pages 1631-1636, XP055635478,
- Jia W W, Mcdermott M, Goldie J, Cynader M, Tan J, Tufaro F: "Selective destruction of gliomas in immunocompetent rats by thymidine kinase-defective herpes simplex virus type 1", Journal of the National Cancer Institute, vol. 86, no. 16, 1994, pages 1209-1215, XP009523451, DOI: 10.1093/jnci/86.16.1209
- SANCHALA, D. S .: "Oncolytic herpes simplex viral therapy: A stride toward selective targeting of cancer cells", Frontiers in Pharmacology, vol. 8, no. 27 0, 2017, pages 1-9, XP055522298,
- GENTRY, B. G.: "Hydroxyurea induces bystander cytotoxicity in cocultures of herpes simplex virus thymidine kinase-expressing and nonexpressing HeLa cells incubated with ganciclovir", Cancer Research, vol. 66, no. 7, 2006, pages 3845-3851, XP055635482,
- LIN, C. Z.: "Advances in the mechanisms of action of cancer-targeting oncolytic viruses", Oncology Letters, vol. 15, no. 4, 2018, pages 4053-4060, XP055635484,
- Lee Jae Woo, Oh Seong Taek, Ahn Chan Hyuk, Lim Kun Woo, Cho Hyun-Il, Kim Gum Ryong, Kim Tai-Gyu: "Antitunor effect of carcinoma cells transduced with herpes simplex virus-thymidine kinase by gancyclovir and radiation", Immune Network, vol. 1, no. 1, 2001, pages 45-52, XP009523564, ISSN: 1598-2629, DOI: 10.4110/in.2001.1.1.45

## Description

### Technical Field

The present invention relates to a pharmaceutical composition and a kit for use in preventing or treating cancer, comprising, as active ingredients, an oncolytic virus and hydroxyurea.

### Background Art

Oncolytic viruses have excellent tumor-specific targeting ability, proliferation ability in cancer cells, and cancer cell-killing ability. Recently, various clinical studies based on oncolytic viruses have been conducted. In 2015, an era of oncolytic virus field began in the United States and Europe, as talimogene laherparepvec (T-Vec), which is an oncolytic virus based on herpes simplex virus, was successfully commercialized as a therapeutic agent for advanced melanoma.

Recently, the usefulness of oncolytic viruses exceeds their own efficacy and the viruses activate tumor immunity, thereby showing their potential as a therapeutic agent that is used in combination with another immunotherapeutic agent. Until 2000 when was an early stage of development of oncolytic viruses, a direct killing effect of the viruses, which is caused by cancer cell-specific proliferation thereof, was relatively more important. However, subsequent clinical studies have found that activation of tumor immunity is a key mechanism rather than a direct cancer cell-killing effect. Based on this finding, therapeutic agents which include an oncolytic virus and an immunotherapeutic agent such as an immune checkpoint inhibitor, both being administered in combination, are recently being developed. This is because it is known that oncolytic viruses convert the tumor microenvironment, in which immunity is suppressed, into a tumor microenvironment appropriate for immunotherapy.

However, so far, in many clinical studies for oncolytic viruses, the tumor microenvironment has been overlooked. In clinical studies, treatment with an oncolytic virus may result in acute tumor necrosis, durable response, or complete response, but in some cases, may lead to a difficult-to-predict result (pharmacodynamics variability) such as progressive disease or early death. By way of illustration, for Pexa-vec based on vaccinia virus, there is a case where a patient died prematurely within one month after treatment with an oncolytic virus in a phase 1 clinical trial.

Therefore, in order to enhance the therapeutic effect of an oncolytic virus, it is necessary to understand interactions between cancer cells, the patient's immune status, and the oncolytic virus; and based on this understanding, there is a need for research on techniques that can increase clinical efficacy of the oncolytic virus.

WO 2017/205674 A1 relates to oncolytic vaccinia virus mutants and using them for cancer treatment. US 2014/234257 A1 relates to oncolytic viruses and methods for treating neoplastic disorders. US 2009/317456 A1 relates to the use of oncolytic viruses and antiangiogenic agents in the treatment of cancer.

### Disclosure of Invention

### Technical Problem

The present inventors studied to enhance the anticancer effect of an oncolytic virus. As a result, the present inventors have found that in a case where the oncolytic virus and hydroxyurea are administered in combination to an individual having cancer, a superior anticancer effect is achieved as compared with a conventional case where only the oncolytic virus is administered, and thus have completed the present invention.

### Solution to Problem

In order to solve the above technical problem, in an aspect of the present invention, there is provided a pharmaceutical composition for use in preventing or treating cancer as defined in claim 1. In another aspect of the present invention, there is provided a kit for use in preventing or treating cancer as defined in claim 5.

### Advantageous Effects of Invention

The pharmaceutical composition of the present invention for use in preventing or treating cancer as defined in the appended claims has superior anticancer effect and safety as compared with a conventional case where only the oncolytic virus is administered, is capable of suppressing growth of cancer cells that are resistant to the oncolytic virus, and is capable of killing cancer cells in which the oncolytic virus can proliferate. Therefore, the pharmaceutical composition of the present invention for use in preventing or treating cancer can be effectively used to treat cancer.

### Brief Description of Drawings

Figures 1, 6, 12 and 16-19 do not relate to embodiments of the invention.
Fig. 1 illustrates results obtained by subjecting 13 cancer cell lines to treatment with an oncolytic virus (OTS-412), and then observing cell viability thereof.
Fig. 2 illustrates results obtained by administering, to mouse-renal cancer cell-implanted mice (Renca I), an oncolytic virus (VV^{tk-}), genetically recombined human granulocyte colony stimulating factor (rhG-CSF), and hydroxyurea (HU), and then measuring the tumor size of the mice.
Fig. 3 illustrates results obtained by administering, to mouse-renal cancer cell-implanted mice (Renca I), an oncolytic virus (VV^{tk-}), rhG-CSF, and HU, and then measuring the body weight of the mice.
Fig. 4 schematically illustrates an experimental schedule for identifying an anticancer therapeutic effect obtained by combined administration of an oncolytic virus (VV^{tk-}) and HU in mouse-renal cancer cell-implanted mice (Renca II).
Fig. 5 illustrates results obtained by administering an oncolytic virus (VV^{tk-}) and HU to mouse-renal cancer cell-implanted mice (Renca II), and then measuring the tumor size of the mice.
Fig. 6 illustrates results obtained by systemically administering an oncolytic virus (WRVV^{tk-}) and HU to mouse-renal cancer cell-implanted mice (Renca III), and then measuring the tumor size of the mice.
Fig. 7 illustrates results obtained by administering an oncolytic virus (OTS-412) and HU to mouse-breast cancer cell-implanted mice (4T1), and then measuring the tumor size of the mice.
Fig. 8 illustrates results obtained by administering an oncolytic virus (OTS-412) and HU to mouse-breast cancer cell-implanted mice (4T1), and then counting the number of nodules appearing on the tumor surface in the mice that were sacrificed 18 days later.
Fig. 9 illustrates results obtained by administering an oncolytic virus (OTS-412) and HU to mouse-breast cancer cell-implanted mice (4T1), and then measuring changes in body weight for 21 days.
Fig. 10 illustrates results obtained by administering an oncolytic virus (OTS-412) and high-dose HU to mouse-breast cancer cell-implanted mice (4T1), and then measuring the tumor size.
Fig. 11 illustrates results obtained by administering an oncolytic virus (OTS-412) and high-dose HU to mouse-breast cancer cell-implanted mice (4T1), and then measuring survival.
Fig. 12 illustrates results obtained by administering an oncolytic virus (OTS-412) and HU to human-lung cancer cell (NCI-H460)-implanted mice, and then measuring the tumor size for 15 days.
Fig. 13 illustrates results obtained by administering an oncolytic virus (OTS-412) and HU to human-colorectal cancer cell (HCT-116)-implanted mice, and then measuring the tumor size for 28 days.
Fig. 14 illustrates results obtained by administering an oncolytic virus (OTS-412) and HU to human-colorectal cancer cell (HCT-116)-implanted mice, and then comparing tumor pictures of the mice in respective groups on day 17 after administration.
Fig. 15 illustrates results obtained by administering an oncolytic virus (OTS-412) and HU to human-colorectal cancer cell (HCT-116)-implanted mice, and then comparing tumor pictures of the mice in respective groups on day 28 after administration.
Fig. 16 illustrates pictures obtained by administering an oncolytic virus (OTS-412) and HU to human-colorectal cancer cell (HT-29)-implanted mice, and then performing H&E staining of the entire mouse tumor.
Fig. 17 illustrates pictures obtained by administering an oncolytic virus (OTS-412) and HU to human-colorectal cancer cell (HT-29)-implanted mice, and then performing TUNEL staining of tumor tissue.
Fig. 18 illustrates results obtained by administering herpes simplex virus 1 (HSV1) and HU to mouse-renal cancer cell-implanted mice, and then measuring the tumor size.
Fig. 19 illustrates results obtained by administering adenovirus and HU to mouse-renal cancer cell-implanted mice, and then measuring the tumor size.

### Best Mode for Carrying out the Invention

Hereinafter, the present invention will be described in detail.

In an aspect of the present invention, there is provided a pharmaceutical composition for use preventing or treating cancer as defined in claim 1. In another aspects, there is provided a kit for use in preventing or treating cancer as defined in claim 5.

The oncolytic virus and hydroxyurea may be administered in combination simultaneously, sequentially, or in reverse order. In some embodiments, the oncolytic virus and hydroxyurea may be administered simultaneously.

As used herein, the term "oncolytic virus" refers to a recombinant virus that destroys cancer cells, the recombinant virus being obtained by manipulating the gene of a virus so that it specifically proliferates only in the cancer cells. The oncolytic virus of use in the invention is derived from a vaccinia virus.

The vaccinia virus may be, but is not limited to, Western Reserve (WR), New York vaccinia virus (NYVAC), Wyeth (The New York City Board of Health; NYCBOH), LC16m8, Lister, Copenhagen, Tian Tan, USSR, Tashkent, Evans, International Health Division-J (IHD-J), or International Health Division-White (IHD-W) vaccinia virus strain.

The oncolytic virus may be a recombinant vaccinia virus (VV^{tk-}) into which granulocyte-macrophage colony-stimulating factor (GM-CSF) and β-galactosidase gene are not inserted and in which thymidine kinase gene is deleted.

In addition, the oncolytic virus may be a recombinant vaccinia virus in which thymidine kinase (TK) gene is deleted and into which human GM-CSF or human G-CSF gene is inserted.

As used herein, the term "thymidine kinase (TK)" refers to an enzyme involved in biosynthesis of nucleotides. TK is an enzyme used for biosynthesis of nucleotides both in cells and viruses. Here, for cells, TK does not exist in normal cells because normal cells do not divide anymore; and even in rapidly dividing cells, such as hair root cells, the amount of TK is not enough for viruses to use. Using these findings, making TK gene defective in a virus allows the virus to proliferate only in a case where TK exists in cancer cells, so that the virus can selectively kill only the cancer cells.

As used herein, the term "GM-CSF" refers to granulocyte-macrophage colony-stimulating factor, a protein secreted by macrophages, T cells, mast cells, natural killer cells, endothelial cells, and fibroblasts. GM-CSF stimulates stem cells to produce granulocytes (neutrophils, basophils, eosinophils) and monocytes. In addition, GM-CSF sharply increases the number of macrophages, and thus induces an immune response. GM-CSF may be of human origin and may be a protein having the sequence of GenBank: AAA52578.1.

As used herein, the term "G-CSF" refers to granulocyte colony-stimulating factor, a cytokine produced by macrophages, fibroblasts, endothelial cells, and the like, upon stimulation caused by inflammation or endotoxin. G-CSF promotes neutrophil production. G-CSF may be of human origin (rhGCSF) and may be a protein having the sequence of GenBank: AAA03056.1.

As used herein, the term "hydroxyurea" refers to a compound having the following formula.

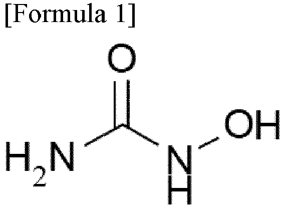

Although the exact mechanism of the hydroxyurea is not elucidated, it is known as an anticancer agent that inhibits DNA synthesis. In addition, the hydroxyurea may be contained in a pharmaceutical composition in the form of a commercially available medicament containing hydroxyurea. The commercially available medicament containing hydroxyurea may be, but is not limited to, Hydroxyurea^{®}, Hydrea^{®}, Droxia^{™}, Mylocel^{™}, Siklos^{®}, or Hydrine Capsule.

A dosage of the oncolytic virus may vary depending on the individual's condition and body weight, severity of disease, type of drug, route and duration of administration, and may be appropriately selected by those skilled in the art. Patients may be administered the oncolytic virus of 1×10⁵ to 1×10¹⁸ virus particles, infectious viral units (TCID50), or plaque forming units (pfu). Specifically, the oncolytic viruses may be administered at a dose of 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶, 1×10¹⁷, or more virus particles, infectious viral units, or plaque forming units, and various numbers and ranges may be included therebetween. Preferably, the oncolytic virus may be administered at a dose of 1×10⁵ to 1×10¹⁰ pfu. More preferably, the oncolytic virus may be administered at a dose of equal to or greater than 1×10⁵ and less than 1×10⁹ pfu. In an embodiment of the present invention, the oncolytic virus was administered at 1×10⁵ or 1×10⁷ pfu.

The hydroxyurea of use in the invention is administered at a dose of 20 mg/kg/day to 90 mg/kg/day. Specifically, the hydroxyurea may be administered at a dose of 20 mg/kg/day to 50 mg/kg/day. In an embodiment of the present invention, the hydroxyurea was administered at 20 mg/kg/day, 30 mg/kg/day, 60 mg/kg/day, or 90 mg/kg/day.

The cancer may be solid cancer or blood cancer. Specifically, the solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary cancer, and pancreatic cancer. In addition, the blood cancer may be any one selected from the group consisting of lymphoma, acute leukemia, and multiple myeloma.

The pharmaceutical composition of the present invention may further comprise a physiologically acceptable carrier. In addition, the pharmaceutical composition of the present invention may further comprise suitable excipients and diluents commonly used in the preparation of pharmaceutical compositions. In addition, the pharmaceutical composition of the present invention may be formulated in the form of an injection according to conventional methods and used.

The pharmaceutical composition may be a preparation for parenteral administration, and include sterile aqueous solutions, non-aqueous solvents, suspensions, emulsions, lyophilized preparations, suppositories, and the like. As the non-aqueous solvents and suspensions, propylene glycol, polyethylene glycol, vegetable oil such as olive oil, injectable ester such as ethyl oleate, and the like may be used. As bases for the suppositories, Witepsol, macrogol, Tween 61, cacao fat, laurin fat, glycerogelatin, and the like may be used.

Regarding route of administration, dosage, and frequency of administration, the pharmaceutical composition may be administered to a subject in various methods and amounts depending on the patient's condition and the presence or absence of side effects; and the optimal administration method, dosage, and frequency of administration may be selected within appropriate ranges by those skilled in the art. In addition, the pharmaceutical composition may be administered in combination with other drugs or physiologically active substances whose therapeutic effect is known for the disease to be treated, or may be formulated in the form of a preparation combined with other drugs.

The pharmaceutical composition may be administered parenterally, such as by an appropriate method including intratumoral, intraperitoneal, subcutaneous, intradermal, intranodal, or intravenous administration. Preferably, the administration may be intratumoral, intraperitoneal, or intravenous administration. On the other hand, a dosage of the pharmaceutical composition may be determined depending on administration schedule, dosage, the patient's health status, and the like.

In another aspect of the present invention, there is provided a kit for preventing or treating cancer as defined in claim 5.

The oncolytic virus is as described above for the pharmaceutical composition.

The second composition comprising hydroxyurea as an active ingredient may be a commercially available medicament. The commercially available medicament including hydroxyurea as an active ingredient may be Hydroxyurea^{®}, Hydrea^{®}, Droxia^{™}, Mylocel^{™}, Siklos^{®}, or Hydrine Capsule.

A dosage of the oncolytic virus may vary depending on the individual's condition and body weight, severity of disease, type of drug, route and duration of administration, and may be appropriately selected by those skilled in the art. Patients may be administered the oncolytic virus of 1×10⁵ to 1×10¹⁸ virus particles, infectious viral units (TCID50), or plaque forming units (pfu). Specifically, the oncolytic viruses may be administered at a dose of 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶, 1×10¹⁷, or more virus particles, infectious viral units, or plaque forming units, and various numbers and ranges may be included therebetween. Preferably, the oncolytic virus may be administered at a dose of 1×10⁵ to 1×10¹⁰ pfu. More preferably, the oncolytic virus may be administered at a dose of equal to or greater than 1×10⁵ and less than 1×10⁹ pfu. In an embodiment of the present invention, the first composition was administered at 1×10⁵ or 1×10⁷ pfu.

In addition, the second composition comprising hydroxyurea is administered at a dose of 20 mg/kg/day to 90 mg/kg/day. Specifically, the second composition comprising hydroxyurea may be administered at a dose of20 mg/kg/day to 50 mg/kg/day. In an embodiment of the present invention, the second composition comprising hydroxyurea was administered at 20 mg/kg/day, 25 mg/kg/day, 30 mg/kg/day, 60 mg/kg/day, or 90 mg/kg/day.

The cancer may be solid cancer or blood cancer. Specifically, the solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary cancer, and pancreatic cancer. In addition, the blood cancer may be any one selected from the group consisting of lymphoma, acute leukemia, and multiple myeloma.

The first composition and the second composition may further comprise a physiologically acceptable carrier. In addition, the pharmaceutical composition of the present invention may further comprise suitable excipients and diluents commonly used in the preparation of pharmaceutical compositions. In addition, the pharmaceutical composition of the present invention may be formulated in the form of an injection according to conventional methods and used.

The first composition and the second composition may be preparations for parenteral administration, and include sterile aqueous solutions, non-aqueous solvents, suspensions, emulsions, lyophilized preparations, suppositories, and the like. As the non-aqueous solvents and suspensions, propylene glycol, polyethylene glycol, vegetable oil such as olive oil, injectable ester such as ethyl oleate, and the like may be used. As bases for the suppositories, Witepsol, macrogol, Tween 61, cacao fat, laurin fat, glycerogelatin, and the like may be used.

Regarding route of administration, dosage, and frequency of administration, the first composition and the second composition may be administered to a subject in various methods and amounts depending on the patient's condition and the presence or absence of side effects; and the optimal administration method, dosage, and frequency of administration may be selected within appropriate ranges by those skilled in the art. In addition, the pharmaceutical composition may be administered in combination with other drugs or physiologically active substances whose therapeutic effect is known for the disease to be treated, or may be formulated in the form of a preparation combined with other drugs.

The first composition and the second composition may be administered parenterally, such as by an appropriate method including intratumoral, intraperitoneal, subcutaneous, intradermal, intranodal, or intravenous administration. Preferably, the administration may be intratumoral, intraperitoneal, or intravenous administration. On the other hand, dosages of the first composition and the second composition may be determined depending on administration schedule, dosage, the patient's health status, and the like.

In addition, the first composition may be administered twice, and may be administered to an individual at intervals of 7 to 30 days. Specifically, the first composition may be administered at intervals of 7 days, 14 days, 21 days, or 30 days.

When the first composition and the second composition are administered sequentially or in reverse order, the second composition may be continuously administered once a day starting from 3 to 5 days before administration of the first composition, and may be continuously administered once a day for 9 to 28 days starting from 24 hours after administration of the first composition.

As used herein, the term "individual" refers to a person suffering from cancer or having a disease whose condition can be alleviated, inhibited, or treated by administration of the pharmaceutical composition of the present invention.

As used herein, the term "administration" refers to introducing an effective amount of a substance into an individual in an appropriate manner, and administration of the oncolytic virus and hydroxyurea may be achieved via general routes that enable them to reach the target tissue.

In addition, the oncolytic virus and hydroxyurea may be administered in combination with other drugs or physiologically active substances whose therapeutic effect is known for the disease to be treated, or may be formulated in the form of a preparation combined with other drugs.

### Mode for the Invention

Hereinafter, the present invention will be described in detail by way of examples. However, the following examples are only to illustrate the present invention, and the present invention is not limited thereto. Examples I, IV, VIII, X, XI and XII are not embodiments of the invention.

### Preparation Example 1. Production of oncolytic virus

### Preparation Example 1.1. Construction of shuttle plasmid vector

In order to produce an oncolytic virus in which thymidine kinase (TK) gene is deleted, the wild-type vaccinia viruses, NYC Department of Health species (Wyeth strain) and Western Reserve species, were purchased from the American Type Culture Collection (ATCC). For recombination, replacement of the TK site in the wild-type viruses was performed using, as vectors, a shuttle plasmid having firefly luciferase reporter (p7.5 promoter) gene, a shuttle plasmid having firefly luciferase reporter and herpes simplex virus thymidine kinase (HSV1-TK) genes, and a shuttle plasmid having GFP gene.

### Preparation Example 1.2. Production of recombinant vaccinia virus

In order to obtain recombinant viruses, Hela cells (ATCC) were seeded into a 6-well plate at 4×10⁵ cells/well and cultured in EMEM medium containing 10% fetal bovine serum. Subsequently, treatment with the wild-type vaccinia virus at an MOI of 0.05 was performed. After 2 hours, the medium was replaced with EMEM medium containing 2% fetal bovine serum, and then transfection with 4 µg of the shuttle plasmid vector, which is constructed in Preparation Example 1.1. and linearized, was performed using XfectTM polymer (Clonetech 631317, USA). After culture for 4 hours, the medium was replaced with EMEM medium containing 2% fetal bovine serum, and then the cells were further cultured for 72 hours. Finally, the infected cells were collected, and then freezing and thawing were repeated three times. Then, the cells were lysed by ultrasonic pulverization, and recombinant vaccinia viruses isolated by a sucrose cushion method were obtained. The viruses were designated VV^{tk-}, WRVV^{tk-}.

Thereafter, in order to obtain recombinant vaccinia viruses containing mutated HSV1-TK gene, mutation of HSV1-TK was induced in TK- osteosarcoma (osteosarcoma 143 TK-) cell line, in the presence of BrdU (thymidine analogue, 15 µg/mL), through 10 successive passages under a biochemical environment (TK-selection pressure) which allows for selection of cells having no TK function. A request for amino acid sequencing of the mutated vaccinia virus was made to Macrogen.

As a result, it was identified that the codon (caa) encoding glutamine (Gln), an amino acid at the 46^{th} position in the C-terminus of HSV1-TK of the mutated vaccinia virus, had point-mutated into a stop codon. In addition, it was identified that the amino acid residues after the 46^{th} position in the C-terminus of HSV1-TK of the mutated vaccinia virus have been deleted. Finally, a mutated vaccinia virus (OTS-412) expressing an HSV1-TK fragment, for which genetic stability is ensured, was obtained.

### I. Cytotoxicity experiment of oncolytic virus (OTS-412)

### Experimental Example 1. Identification of tumor cell-killing effect of oncolytic virus

In order to identify cytotoxicity of the oncolytic virus (OTS-412), toxicity was evaluated in 10 human cancer cell lines and 3 mouse cancer cell lines. Specifically, toxicity was evaluated in HeLa, PC-3, DU-145, HT-29, HCT-116, A549, NCI-H23, NCI-H460, MCF-7, MDA-MB-231, 4T1, Renca, and B16F10 cancer cell lines. HeLa, A549, 4T1, and B16F10 cells were obtained from ATCC (USA), and the remaining nine cancer cell lines were obtained from the Korea Cell Line Bank (KCLB).

First, each cancer cell line was infected with the oncolytic virus at an MOI of 0.5 (0.5 pfu/cell) and then cultured for 72 hours. Subsequently, cytotoxicity was analyzed using Cell Counting Kit8 (CCK8).

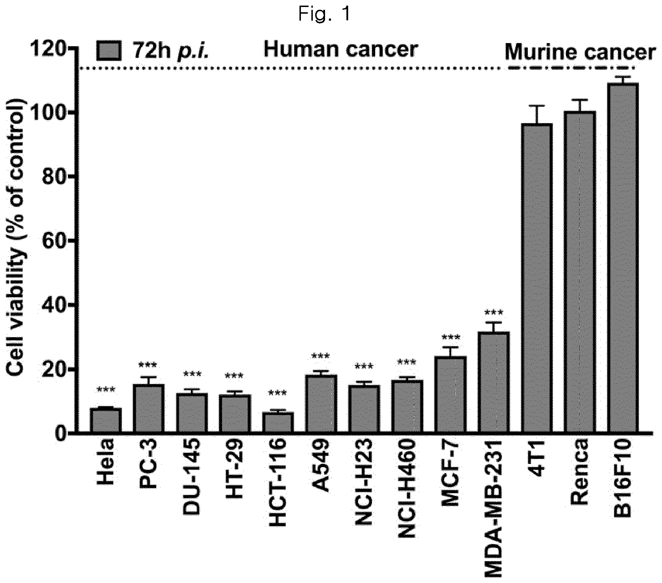

As a result, 4T1, Renca, and B16F10 cancer cell lines, which are mouse cancer cell lines, showed viability of 80% or more and showed relatively high resistance. However, it was identified that most of the remaining cancer cell lines showed viability of 30% or less after 72 hours and showed high cytotoxicity (Fig. 1). From this, it was identified that the oncolytic virus hardly proliferated in the mouse cancer cell lines.

Furthermore, animal experiments were performed by implanting human-derived or mouse-derived cancer cell lines, which had different oncolytic virus proliferative capacity, into mice, respectively, so that human cancer cell-implanted mice (xenograft model) and mouse cancer cell-implanted mice (allograft model) were produced. In particular, for the mouse cancer cell-implanted mice, an experiment was designed to identify whether the oncolytic virus had an increased anticancer effect in a case of being administered in combination with hydroxyurea in a state where proliferation of the oncolytic virus was limited.

### II. Identification of anticancer therapeutic effect of oncolytic virus (VV^{tk-}) and hydroxyurea in mouse renal cancer cell-implanted mice: Renca I

### Example 2.1 Production of mouse renal cancer cell-implanted mice and drug administration

Balb/c mice (female, 7-week-old) supplied by Orient Bio (Busan, Korea) were acclimatized for a week and then allografted with Renca cancer cell line (Korea Cell Line Bank) at 5×10⁶ cells. Observation was made until the tumor size reached 100 mm³ to 150 mm³, and then administration of the oncolytic virus began. On the other hand, the vaccinia virus-derived oncolytic virus (VV^{tk-}) hardly proliferated in a mouse renal cancer cell-implanted mouse model.

The above-produced mouse renal cancer cell-implanted mice were divided into 4 groups (n=4). The group receiving saline intratumorally was set as a negative control group, and the group receiving oncolytic virus (VV^{tk-}, 1×10⁷ pfu) was set as a positive control group. In addition, the group receiving oncolytic virus (VV^{tk-}, 1×10⁷ pfu) and recombinant human granulocyte colony-stimulating factor (rh-G-CSF, 75 µg/kg), and the group receiving oncolytic virus (VV^{tk-}, 1×10⁷ pfu) and hydroxyurea (30 mg/kg) were set as experimental groups. The oncolytic virus was administered intratumorally, and its second administration was made 15 days after the first administration. rh-G-CSF or hydroxyurea was administered intraperitoneally starting from 4 days before administration of the oncolytic virus until sacrifice.

### Example 2.2. Identification of changes in tumor size

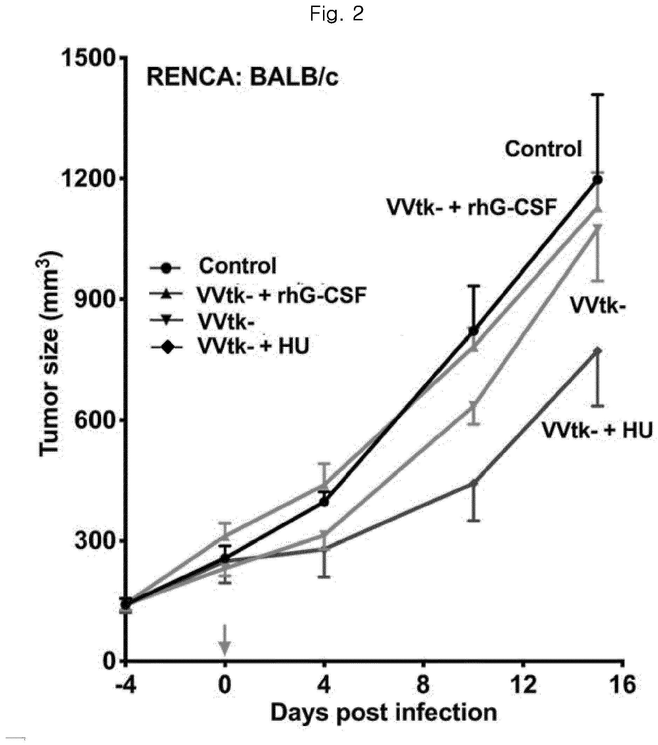

The mice in respective groups of Example 2.1 were administered drugs. Then, the mice were sacrificed on day 16 and the tumor size was measured. As a result, it was observed that the group receiving oncolytic virus and rh-G-CSF showed a similar tumor size to the negative control group and the positive control group, and that the tumor size had increased 10 times or more as compared with the initial one. On the other hand, it was observed that in the group receiving oncolytic virus and hydroxyurea in combination, the tumor size of the mice was smaller as compared with the negative control group, the positive control group, and other experimental groups, and that the tumor size had increased about 7 times as compared with the initial one (Fig. 2). In particular, from the viewpoint that the tumor size was small as compared with the positive control group, it was identified that in a case where the hydroxyurea and oncolytic virus were administered in combination, an increased anticancer effect was achieved.

### Example 2.3 Identification of changes in body weight

Body weights of the mice were measured before administration of respective drugs to the control groups and the experimental groups of Example 2.1, on the day of administration, and on days 4, 10, and 15 after administration. The mouse body weight was calculated by subtracting a tumor weight from a body weight on day 18, and the tumor weight (v) was calculated as v = x²y/2 wherein x and y are the shortest and longest diameters, respectively.

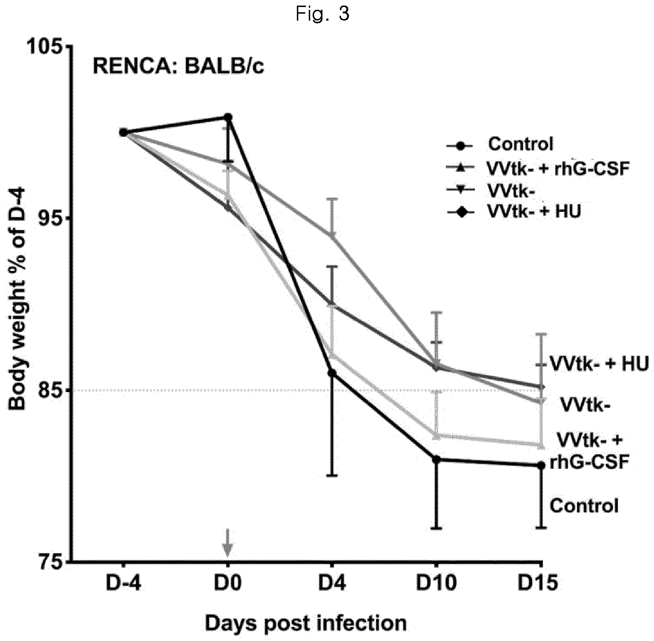

As a result, it was identified that the body weight of the mice in the negative control group and the group receiving oncolytic virus and rh-G-CSF decreased by about 20% or more, while the body weight of the mice in the experimental groups remained stable at 85% or more of the body weight before drug administration (Fig. 3).

### III. Identification of anticancer therapeutic effect of oncolytic virus (VV^{tk-}) and hydroxyurea in mouse renal cancer cell-implanted mice: Renca II

### Example 3.1. Production of mouse renal cancer cell-implanted mice and drug administration

Balb/c mice (female, 7-week-old) supplied by Orient Bio (Busan, Korea) were acclimatized for a week and then allografted with Renca cancer cell line (Korea Cell Line Bank) at 5×10⁶ cells. Observation was made until the tumor size reached 100 mm³ to 150 mm³, and then administration of the oncolytic virus began. On the other hand, the vaccinia virus-derived oncolytic virus (VV^{tk-}) hardly proliferated in a mouse renal cancer cell-implanted mouse model.

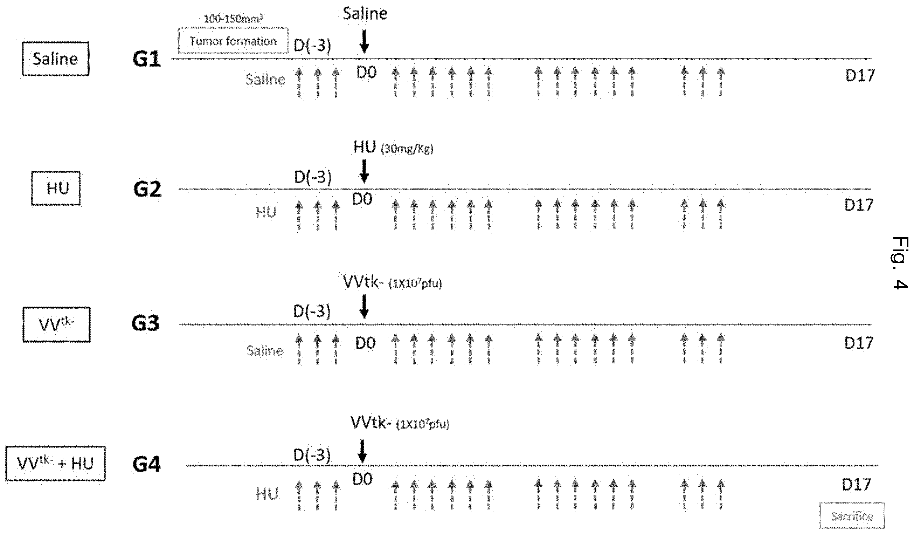

The above-produced mouse renal cancer cell-implanted mice were divided into 4 groups (n=13). The group receiving saline intratumorally was set as a negative control group, and the group receiving oncolytic virus (VV^{tk-}, 1×10⁷ pfu) or hydroxyurea (30 mg/kg) was set as a positive control group. The group receiving oncolytic virus and hydroxyurea in combination was set as an experimental group. The oncolytic virus was administered intratumorally once, and its second administration was made 17 days after the first administration. The hydroxyurea was administered intraperitoneally once a day starting from 3 days before administration of the oncolytic virus until 1 day before sacrifice, except for the day of administration of the oncolytic virus (Fig. 4).

### Example 3.2. Identification of changes in tumor size

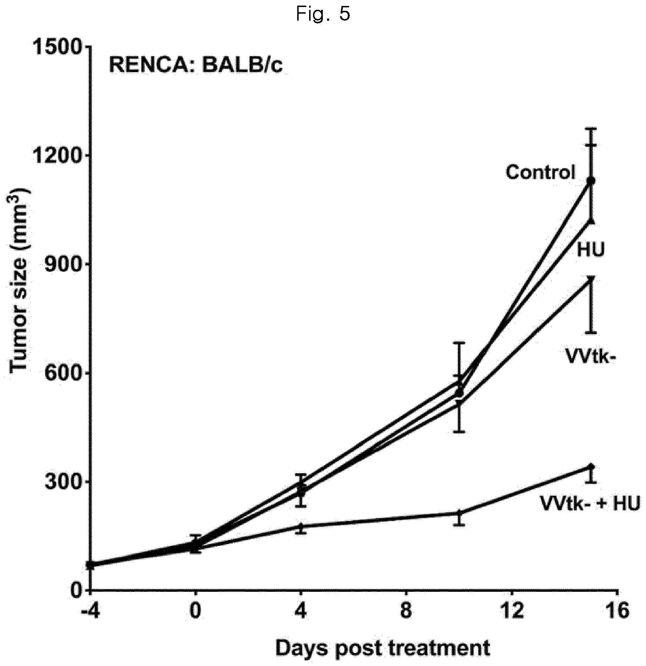

The mice in respective groups of Example 3.1 were administered drugs. Then, the mice were sacrificed on day 17 and the tumor size was measured. As a result, it was identified that the tumor size of the mice in the negative control group and the positive control group had rapidly increased by 10 to 15 times, while the tumor size of the mice in the experimental group was nearly 3 times smaller than the negative control group and the positive control group (Fig. 5). From this, it was identified that in a case where the oncolytic virus and hydroxyurea were administered in combination, a greatly increased anticancer effect was achieved even in a tumor model in which the oncolytic virus hardly proliferated.

### IV. Identification of anticancer therapeutic effect of oncolytic virus (WRVV^{tk-}) and hydroxyurea in mouse renal cancer cell-implanted mice: Renca III

### Example 4.1. Production of mouse renal cancer cell-implanted mice and drug administration

Balb/c mice (female, 7-week-old) supplied by Orient Bio (Busan, Korea) were acclimatized for a week and then allografted with Renca cancer cell line (Korea Cell Line Bank) at 5×10⁶ cells. Observation was made until the tumor size reaches 100 mm³ to 150 mm³, and then administration of the oncolytic virus began. On the other hand, the Western reserve species vaccinia virus-derived oncolytic virus (WRVV^{tk-}) can proliferate in a mouse renal cancer cell-implanted mouse model.

The above-produced mouse renal cancer cell-implanted mice were divided into 4 groups (n=6). The group receiving saline intratumorally was set as a negative control group, and the group receiving oncolytic virus (WRVV^{tk-}, 1×10⁷ pfu) or hydroxyurea (60 mg/kg) was set as a positive control group. The group receiving oncolytic virus and hydroxyurea in combination was set as an experimental group. The oncolytic virus was administered intraperitoneally once. The hydroxyurea was administered intraperitoneally once a day starting from 1 day before administration of the oncolytic virus until day 6 after administration, except for the day of administration of the oncolytic virus.

### Example 4.2. Identification of changes in tumor size

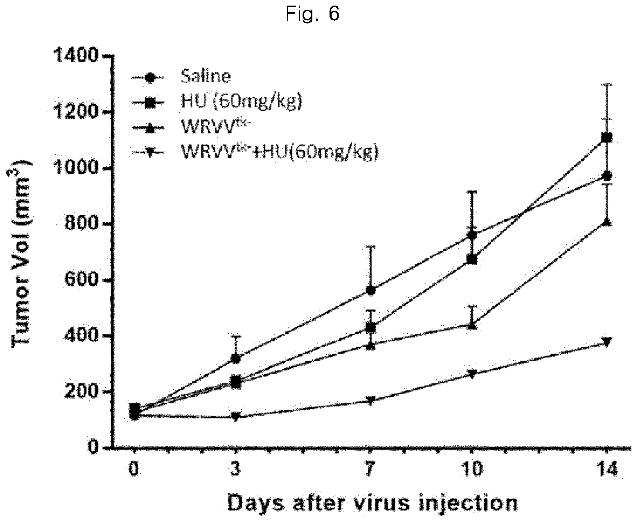

The mice in respective groups of Example 4.1 were administered drugs. Then, the tumor size was measured for 14 days. As a result, it was identified that the tumor size of the mice in the negative control group and the positive control group had increased by nearly 8 to 10 times, while the tumor size of the mice in the experimental group had increased by nearly 3 times. From this, it was identified that tumor growth was remarkably suppressed in a case where the oncolytic virus and hydroxyurea were administered in combination (Fig. 6).

### V. Identification of anticancer therapeutic effect of oncolytic virus (OTS-412) and hydroxyurea in mouse breast cancer cell-implanted mice: 4T1 I

### Example 5.1. Production of mouse breast cancer cell-implanted mice and drug administration

Balb/c mice (female, 7-week-old) supplied by Orient Bio (Busan, Korea) were acclimatized for a week and then allografted with 4T1 cancer cell line (Korea Cell Line Bank) at 2×10⁶ cells. Observation was made until the tumor size reached 100 mm³ to 150 mm³, and then administration of the oncolytic virus began. On the other hand, the vaccinia virus-derived oncolytic virus (OTS-412) hardly proliferates in a mouse breast cancer cell-implanted mouse model.

The above-produced mouse breast cancer cell-implanted mice were divided into 4 groups (n=4). The group receiving saline intratumorally was set as a negative control group, and the group receiving oncolytic virus (OTS-412, 1×10⁷ pfu) or hydroxyurea (30 mg/kg) was set as a positive control group. The group receiving oncolytic virus and hydroxyurea in combination was set as an experimental group. The oncolytic virus was administered intratumorally once. The hydroxyurea was administered intraperitoneally once a day starting from 3 days before administration of the oncolytic virus until 1 day before sacrifice, except for the day of administration of the oncolytic virus.

### Example 5.2. Identification of changes in tumor size

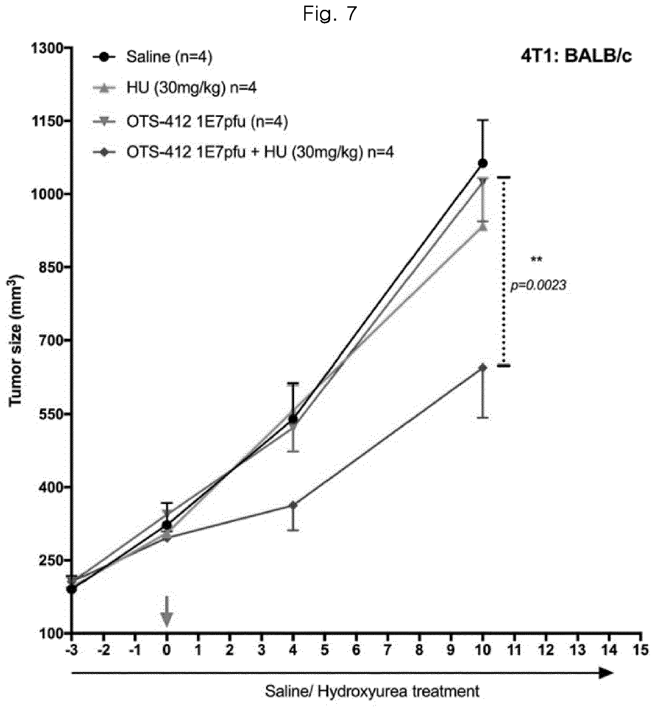

The mice in respective groups of Example 5.1 were administered drugs. Then, changes in tumor size were measured for 10 days. As a result, it was observed that the tumor size of the mice in the negative control group and the positive control group had increased by nearly 5 times, while the tumor size of the mice in the experimental group had increased by nearly 3 times (Fig. 7). From this, it was identified that in a case where the oncolytic virus and hydroxyurea were administered in combination, a superior anticancer effect was achieved as compared with a case where each drug was administered alone.

### VI. Identification of anticancer therapeutic effect of oncolytic virus (OTS-412) and hydroxyurea in mouse breast cancer cell-implanted mice: 4T1 II

### Example 6.1. Production of mouse breast cancer cell-implanted mice and drug administration

Balb/c mice (female, 7-week-old) supplied by Orient Bio (Busan, Korea) were acclimatized for a week and then allografted with 4T1 cancer cell line (Korea Cell Line Bank) at 1×10⁶ cells. Observation was made until the tumor size reached 100 mm³ to 150 mm³, and then administration of the oncolytic virus began. On the other hand, the vaccinia virus-derived oncolytic virus (OTS-412) hardly proliferates in a breast cancer cell line-implanted mouse model. In addition, the 4T1 cell line-implanted mice are an animal model in which metastasis to the whole body, including lung tissue, progresses; and in general, the metastasis is evaluated by the number of nodules on the tumor surface.

The above-produced mouse breast cancer cell-implanted mice were divided into 4 groups (n=5). The group receiving saline intratumorally was set as a negative control group, and the group receiving oncolytic virus (OTS-412, 1×10⁷ pfu) or hydroxyurea (30 mg/kg) was set as a positive control group. The group receiving oncolytic virus and hydroxyurea in combination was set as an experimental group. For the oncolytic virus, its second administration was made 7 days after the first intratumoral administration. The hydroxyurea was administered intraperitoneally once a day starting from 3 days before administration of the oncolytic virus until 3 days before sacrifice, except for the day of administration of the oncolytic virus.

### Example 6.2. Identification of changes in tumor size

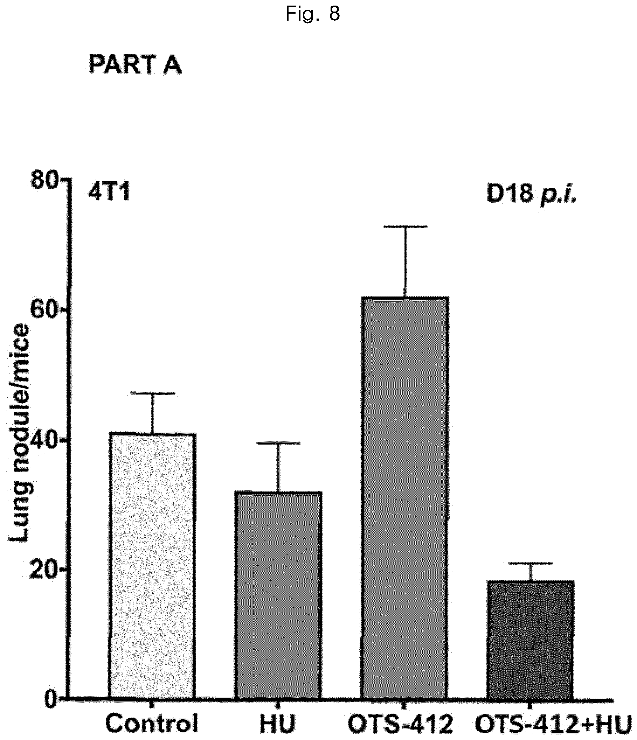

The mice in respective groups of Example 6.1 were administered drugs. Then, the mice were sacrificed on day 21 and the number of tumor nodules was counted. As a result, for the mice in the group receiving only oncolytic virus, the number of nodules was 1.5 times higher than that of the negative control group. On the other hand, for the mice of the experimental group, it was identified that the number of nodules produced was 0.5 times which was remarkably small (Fig. 8). From this, it was identified that in a case where the oncolytic virus and hydroxyurea were administered in combination, metastasis to other organs was suppressed.

### Example 6.3. Identification of safety following repeated administration

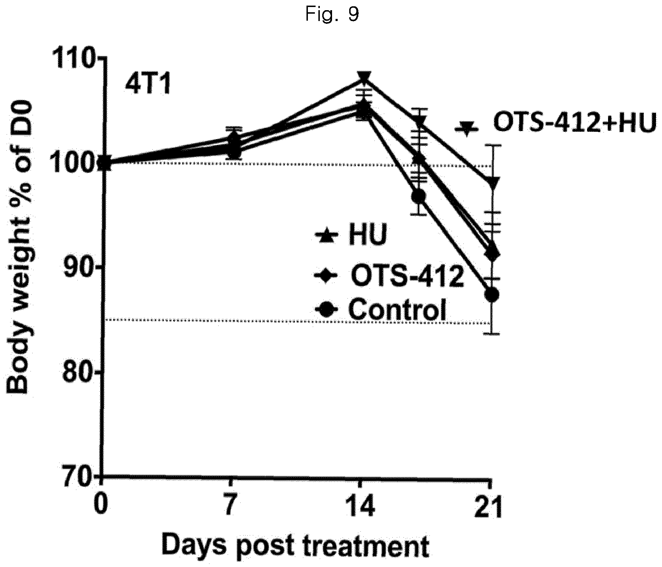

A mouse breast cancer cell-implanted mouse model as in Example 6.1 was produced (n=10) and administered drugs. Then, body weights of the mice were measured on days 7, 14, 17, and 21 after drug administration. As a result, the body weight of the mice in all groups tended to decrease after 2 weeks; however, the highest body weight was observed on day 21 for the mice in the experimental group. Even for the mice in the other three groups, body weight loss of around 10% was observed on day 21. From this, it was identified that safety was ensured even in a case where the oncolytic virus was repeatedly administered and was administered in combination with hydroxyurea (Fig. 9).

### VII. Identification of anticancer therapeutic effect of oncolytic virus (OTS-412) and high-dose hydroxyurea in mouse breast cancer cell-implanted mice: 4T1 III

### Example 7.1. Production of mouse breast cancer cell-implanted mice and drug administration

Balb/c mice (female, 7-week-old) supplied by Orient Bio (Busan, Korea) were acclimatized for a week and then allografted with 4T1 cancer cell line (Korea Cell Line Bank) at 5×10⁶ cells. Observation was made until the tumor size reached 50 mm³ to 200 mm³, and then administration of the oncolytic virus began. On the other hand, the vaccinia virus-derived oncolytic virus (OTS-412) hardly proliferates in a breast cancer cell-implanted mouse model.

The above-produced mouse breast cancer cell-implanted mice were divided into 4 groups. The group receiving saline intratumorally was set as a negative control group, and the group receiving oncolytic virus (OTS-412, 1×10⁷ pfu) or high-dose hydroxyurea (90 mg/kg) was set as a positive control group. The group receiving oncolytic virus and high-dose hydroxyurea in combination was set as an experimental group. The oncolytic virus was administered intratumorally once. The hydroxyurea was administered intraperitoneally once a day starting from 3 days before administration of the oncolytic virus until 1 day before sacrifice, except for the day of administration of the oncolytic virus.

### Example 7.2. Identification of changes in tumor size

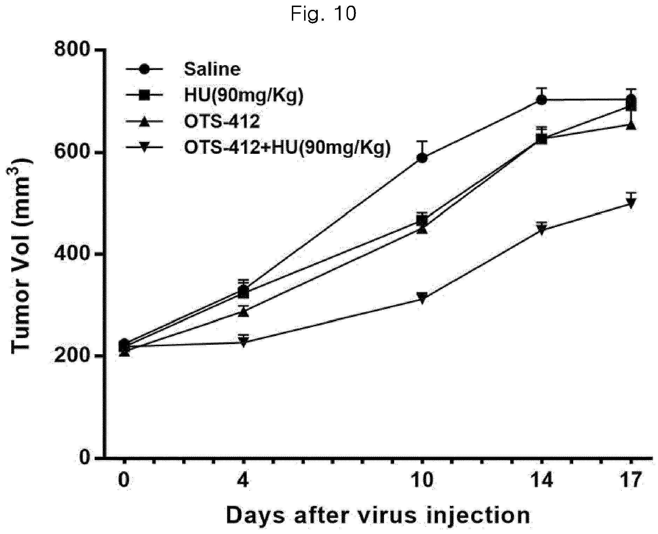

The mice in respective groups of Example 7.1 were administered drugs. Then, the tumor size was measured for 17 days. As a result, it was identified that the tumor size of the mice in the negative control group and the positive control group had increased by nearly 3.5 times, while the tumor size of the mice in the experimental group had increased by 2.5 times. From this, it was identified that in a case where the oncolytic virus and hydroxyurea were administered in combination, tumor growth was suppressed, as compared with a case where the oncolytic virus or hydroxyurea was administered alone (Fig. 10).

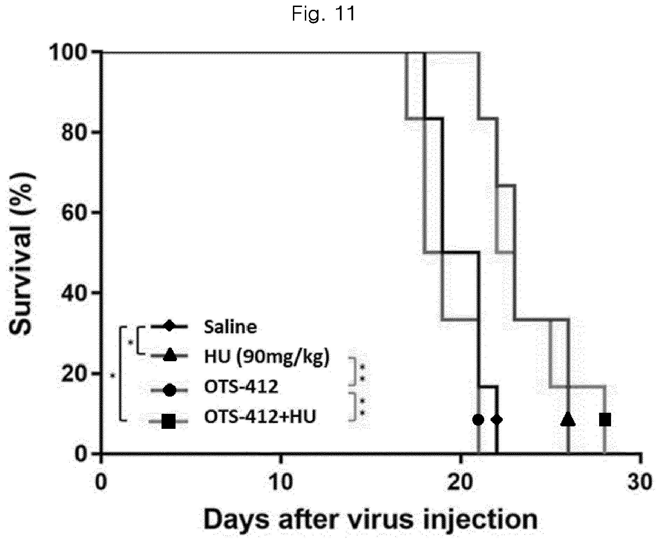

In addition, survival of the mice in respective groups until day 28 was checked. As a result, all mice in the group receiving only oncolytic virus died on day 21, and 1 mouse survived for the negative control group. On the other hand, it was identified that the mice had survived to the very end in the group receiving oncolytic virus and hydroxyurea in combination (Fig. 11). From this, safety and anticancer effect were confirmed for combined administration of high-dose hydroxyurea and oncolytic virus.

### VIII. Identification of anticancer therapeutic effect of oncolytic virus (OTS-412) and hydroxyurea in human lung cancer cell (NCI-H460)-implanted mice

### Example 8.1. Production of human lung cancer cell-implanted mice and administration

Balb/c nu/nu mice supplied by Orient Bio (Busan, Korea) were acclimatized for a week and then allografted with NCI-H460 lung cancer cells (Korea Cell Line Bank) at 1×10⁶ cells. When the tumor size reached 300 mm³ to 400 mm³, administration of the oncolytic virus began. The vaccinia virus-derived oncolytic virus (OTS-412) can proliferate in a lung cancer cell line-implanted mouse model.

The above-produced human lung cancer cell-implanted mice were divided into 4 groups. The group receiving saline intratumorally was set as a negative control group, and the group receiving hydroxyurea or oncolytic virus (OTS-412, 1×10⁵ pfu) was set as a positive control group. The group receiving oncolytic virus and hydroxyurea (20 mg/kg) intraperitoneally was set as an experimental group. The oncolytic virus was administered twice, and its second administration was made 5 days after the first administration. The hydroxyurea was administered once daily starting from the day before the first administration of oncolytic virus until the day of sacrifice, except for the day of administration of the oncolytic virus.

### Example 8.2. Identification of changes in tumor size

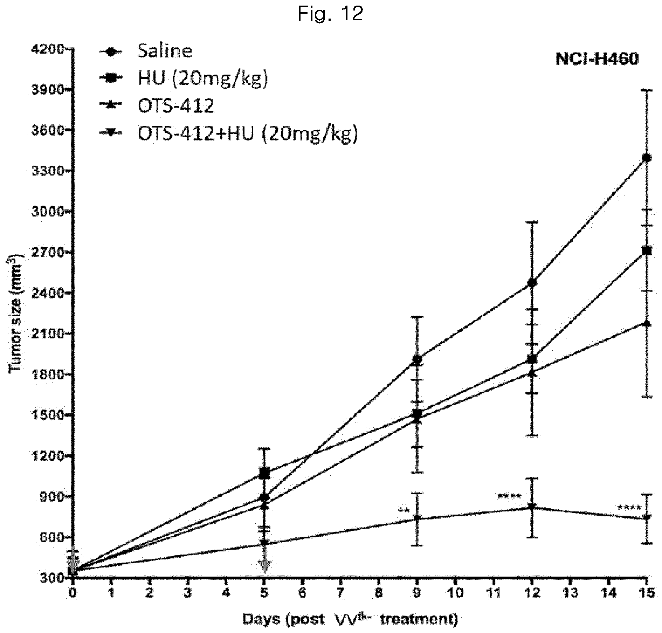

The mice in respective groups of Example 8.1 were administered drugs. Then, the mice were sacrificed on day 15 and the tumor size was measured. As a result, it was observed that the tumor size of the mice in the negative control group and the positive control groups had rapidly increased by 11, 9 and 7 times, respectively. On the other hand, the tumor size of the mice in the experimental group increased by about 2.5 times, and tended to decrease starting from day 12 (Fig. 12). From this, it was identified that in a case where the oncolytic virus and hydroxyurea were administered in combination, tumor growth was effectively suppressed, as compared with a case where only the oncolytic virus was administered alone.

### IX. Identification of anticancer therapeutic effect of oncolytic virus (OTS-412) and hydroxyurea in human colorectal cancer cell (HCT-116)-implanted mice

### Example 9.1. Production of human colorectal cancer cell-implanted mice and administration

Balb/c nu/nu mice (female, 7-week-old) supplied by Orient Bio (Busan, Korea) were acclimatized for a week and then allografted with HCT-116 cancer cell line (Korea Cell Line Bank), a human colorectal cancer cell line, at 2.5×10⁶ cells. Observation was made until the tumor size reached 20 mm³ to 250 mm³, and then administration of the oncolytic virus began.

The above-produced human colorectal cancer cell-implanted mice were divided into 4 groups (n=5). The group receiving saline intratumorally was set as a negative control group, and the group receiving hydroxyurea or oncolytic virus (OTS-412, 1×10⁷ pfu) was set as a positive control group. The group receiving oncolytic virus and hydroxyurea (30 mg/kg) intraperitoneally was set as an experimental group. The oncolytic virus was administered once, and the hydroxyurea was administered once daily starting from 3 days before administration of the oncolytic virus until the day of sacrifice, except for the day of administration of the oncolytic virus. The vaccinia virus-derived oncolytic virus (OTS-412) can proliferate in a colorectal cancer cell line-implanted mouse model.

### Example 9.2. Identification of changes in tumor size

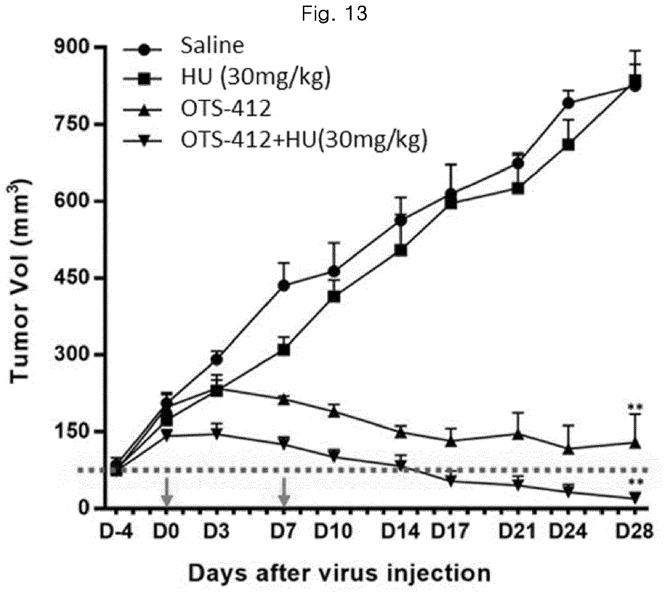

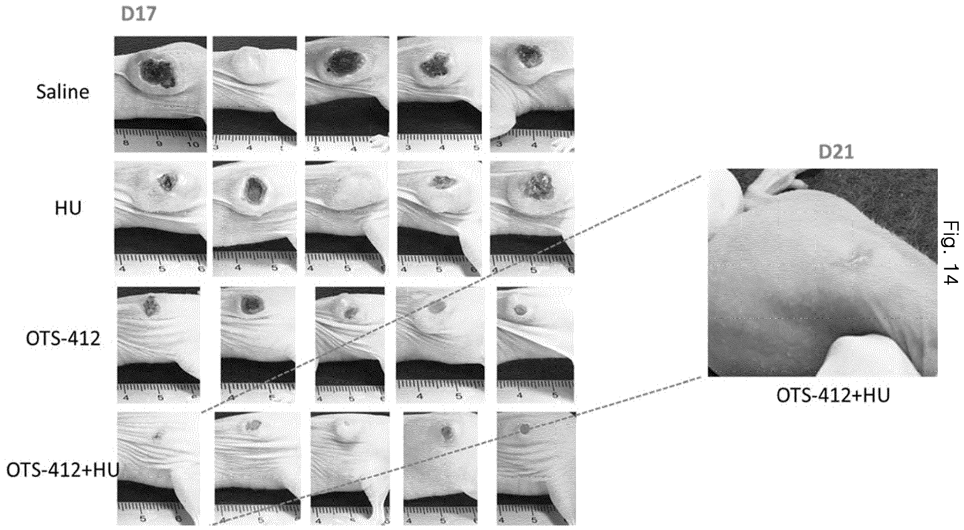

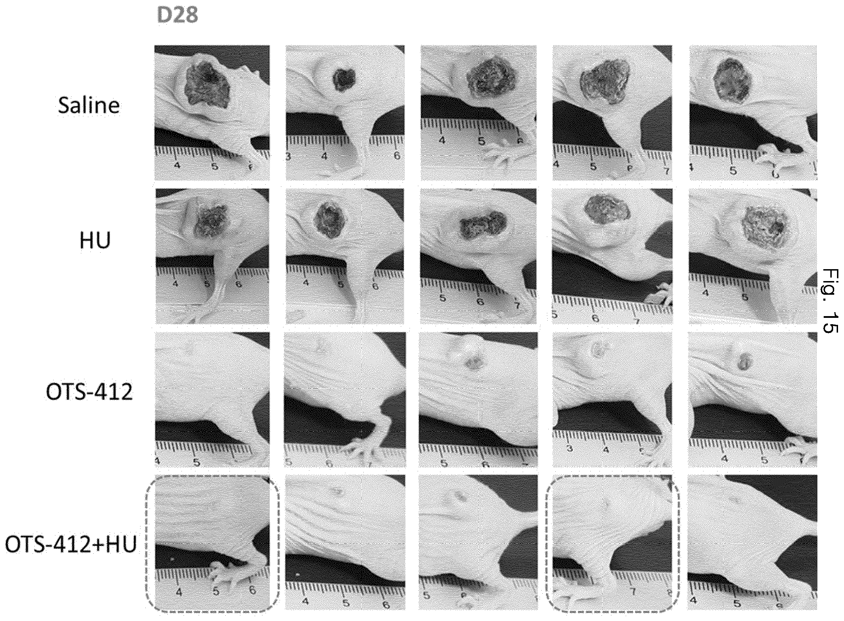

The mice in respective groups of Example 9.1 were administered drugs. Then, the mice were sacrificed on day 28 and the tumor size was measured. As a result, it was observed that the tumor size of the mice in the negative control group and the group receiving only hydroxyurea had sharply increased by 11 times. It was identified that in the group receiving oncolytic virus alone, the tumor size gradually decreased starting from 3 days after administration of the oncolytic virus and remained at the initial tumor size. On the other hand, for the experimental group, it was identified that the tumor size gradually decreased starting from the day of administration of the oncolytic virus, began to decrease, as compared with the initial tumor size, on day 14 after administration of the oncolytic virus, and almost disappeared on day 28 (Fig. 13). In particular, complete remission was observed on day 28 for 2 animals in the experimental group (Figs. 14 and 15).

### X. Identification of anticancer therapeutic effect of oncolytic virus (VV^{tk-}) and hydroxyurea in human colorectal cancer cell (HT-29)-implanted mice

### Example 10.1 Production of human colorectal cancer cell (HT-29)-implanted mice and administration

Balb/c nu/nu mice (female, 7-week-old) supplied by Orient Bio (Busan, Korea) were acclimatized for a week and then allografted with HT-29 cancer cell line (Korea Cell Line Bank), a human colorectal cancer cell line, at 5×10⁶ cells. Observation was made until the tumor size reached 150 mm³ to 200 mm³, and then administration of the oncolytic virus began.

The above-produced human colorectal cancer cell-implanted mice were divided into the following 2 groups, and experiments were performed for 8 weeks: the group receiving only oncolytic virus (OTS-412, 1×10⁷ pfu) and the group additionally receiving hydroxyurea (25 mg/kg) intraperitoneally. From week 1 to week 4, the oncolytic virus was administered to each group twice a week. At week 5 and week 7, the oncolytic virus was administered once a week; and at week 6 and week 8, no oncolytic virus was administered to both groups. The hydroxyurea was administered once a day from week 6 to week 8. On the other hand, the vaccinia virus-derived oncolytic virus (VV^{tk-}) can proliferate in a colorectal cancer cell line-implanted mouse model.

### Example 10.2. Identification of tumor size and tumor tissue apoptosis

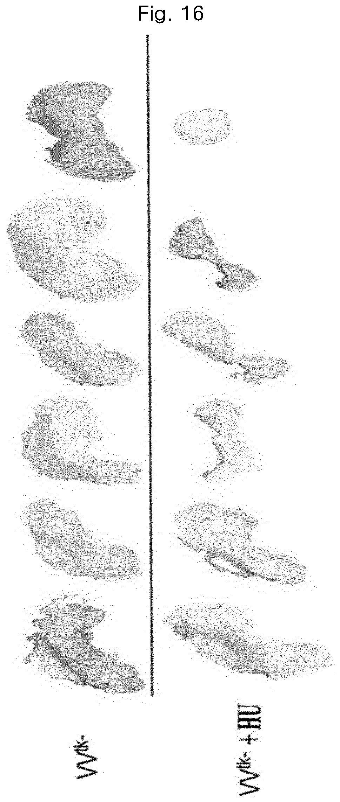

On the last day of week 8, the mice in respective groups were sacrificed and the tumor size was measured. As a result, it was identified that in the group receiving oncolytic virus and hydroxyurea, the tumor size decreased and the condition of tumor tissue was ameliorated, as compared with the group receiving only oncolytic virus (Fig. 16).

In addition, H&E staining of the collected tumor tissue was performed using hematoxylin and eosin. Here, a darkly H&E-stained portion indicates viable cells. As a result, it was identified that the group receiving oncolytic virus and hydroxyurea showed a smaller overall tumor size and a smaller darkly stained portion than the group receiving only oncolytic virus (Fig. 16). That is, it was identified that the experimental group receiving oncolytic virus and hydroxyurea in combination showed a superior apoptotic effect, as compared with the control group receiving only oncolytic virus.

In addition, the collected tumor tissue was analyzed by performing H&E staining and TUNEL staining. Here, a darkly H&E-stained portion indicates viable cells; and in fluorescence staining, the oncolytic virus has red fluorescence and the dead cell has green fluorescence.

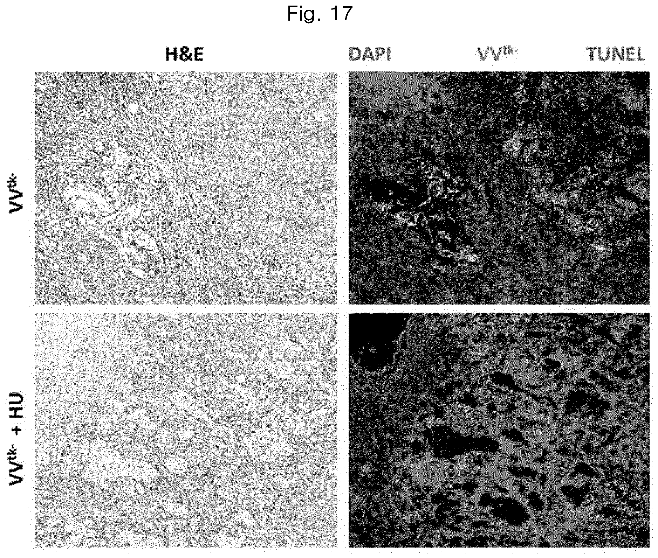

As a result, in the tumor tissue of the group receiving oncolytic virus and hydroxyurea in combination, more dead cells were observed than the group receiving only oncolytic virus. In addition, for the control group, the dead cells matched the oncolytic viruses in terms of stained portion, which identified cancer cell death caused by the oncolytic virus. On the other hand, it was identified that in the group receiving oncolytic virus and hydroxyurea in combination, the portion of dead cells was different from the stained portion of the oncolytic viruses. This identifies that in the group receiving oncolytic virus and hydroxyurea in combination, apoptosis occurred even in cancer cells that were not infected with the oncolytic virus (Fig. 17).

### XI. Identification of anticancer therapeutic effect of herpes simplex virus (HSV1) and hydroxyurea in mouse renal cancer cell-implanted mice

### Example 11.1 Production of mouse renal cancer cell (Renca)-implanted mice and administration

Balb/c mice (female, 7-week-old) supplied by Orient Bio (Busan, Korea) were acclimatized for a week and then allografted with Renca cancer cell line (Korea Cell Line Bank), a mouse renal cancer cell line, at 5×10⁶ cells. Observation was made until the tumor size reaches 50 mm³ to 200 mm³, and then administration of the oncolytic virus began.

The above-produced mouse renal cancer cell-implanted mice were divided into 4 groups. The group receiving saline intratumorally was set as a control group, and the group receiving hydroxyurea or herpes simplex virus (HSV1, 1×10⁶ pfu) was set as a positive control group. The group receiving HSV1 and hydroxyurea (30 mg/kg) was set as an experimental group. HSV1 was administered intratumorally once 3 days after administration of the hydroxyurea, and the hydroxyurea was administered intraperitoneally 6 times a week for 20 days after administration of the oncolytic virus.

### Example 11.2. Identification of changes in tumor size

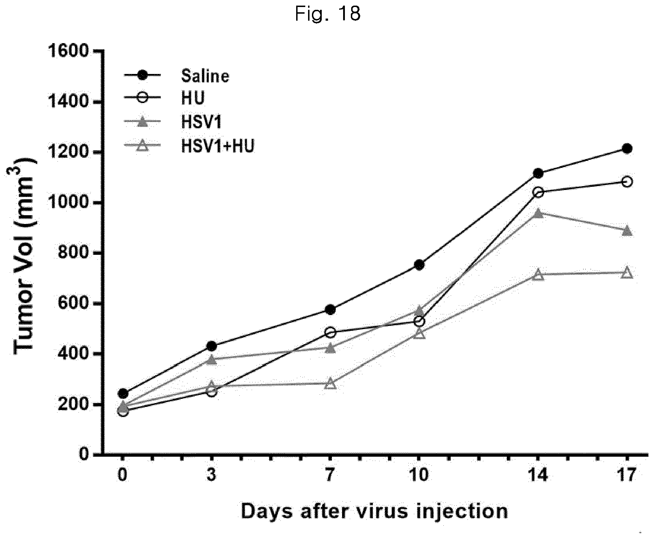

The mice in respective groups of Example 11.1 were administered drugs. Changes in tumor size were measured on the day of drug administration, and days 3, 7, 10, 14, and 17 after administration. As a result, it was identified that the experimental group showed the smallest tumor size. From this, it was identified that in a case where HSV1 and hydroxyurea were administered in combination, tumor growth was more effectively suppressed than a case where only oncolytic virus was administered (Fig. 18).

### XII. Identification of anticancer therapeutic effect of adenovirus and hydroxyurea in mouse renal cancer cell-implanted mice

### Example 12.1. Production of mouse renal cancer cell (Renca)-implanted mice and administration

Balb/c mice (female, 7-week-old) supplied by Orient Bio (Busan, Korea) were acclimatized for a week and then allografted with Renca cancer cell line (Korea Cell Line Bank), a mouse renal cancer cell line, at 5×10⁶ cells. Observation was made until the tumor size reached 50 mm³ to 200 mm³, and then administration of the oncolytic virus began.

The above-produced mouse renal cancer cell-implanted mice were divided into 3 groups. The group receiving saline intratumorally was set as a negative control group, and the group receiving only oncolytic virus (adenovirus, 1×10⁷ pfu) was set as a positive control group. The group receiving adenovirus (1×10⁷ pfu) and hydroxyurea (30 mg/kg) was set as an experimental group. The adenovirus was administered intratumorally once 3 days after administration of the hydroxyurea, and the hydroxyurea was administered intraperitoneally 6 times a week for 16 days after administration of the oncolytic virus.

### Example 12.2. Identification of changes in tumor size

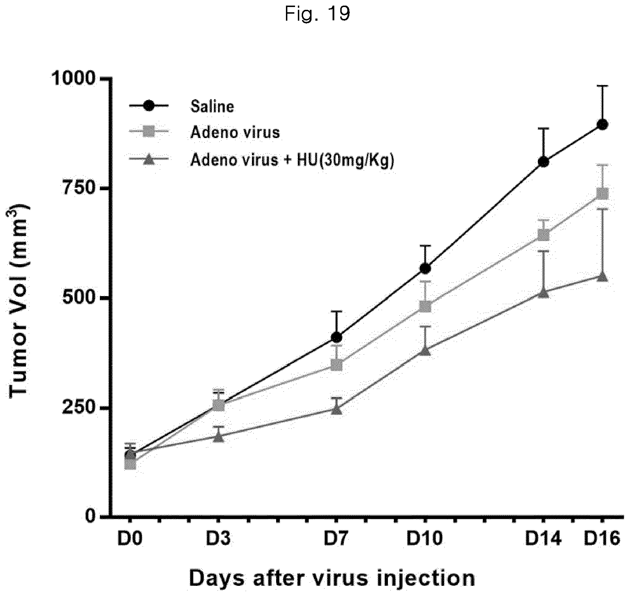

The mice in respective groups of Example 12.1 were administered drugs. Changes in tumor size were measured on the day of drug administration, and days 3, 7, 10, 14, and 17 after administration. As a result, it was identified that the group receiving oncolytic virus and hydroxyurea in combination showed the smallest tumor size. From this, it was identified that in a case where adenovirus and hydroxyurea were administered in combination, tumor growth was more effectively suppressed than a case where only oncolytic virus was administered (Fig. 19).

## Claims

1. A pharmaceutical composition for use in preventing or treating cancer, comprising as active ingredients:
an oncolytic virus, wherein the oncolytic virus is a vaccinia virus in which thymidine kinase gene is deleted,
and hydroxyurea,
wherein the oncolytic virus is administered at a dose of 1×10⁵ pfu or more pfu, and
wherein the hydroxyurea is administered at a dose of 20 mg/kg/day to 90 mg/kg/day.

2. The pharmaceutical composition for use according to claim 1, wherein the oncolytic virus is administered at a dose of 1×10⁵ pfu to 1×10¹⁰ pfu.

3. The pharmaceutical composition for use according to claim 1 or claim 2, wherein the cancer is any one selected from the group consisting of lung cancer, colorectal cancer, prostate cancer, thyroid cancer, breast cancer, brain cancer, head and neck cancer, esophageal cancer, skin cancer, thymic pressure, gastric cancer, colon cancer, liver cancer, ovarian cancer, uterine cancer, bladder cancer, rectal cancer, gallbladder cancer, biliary cancer and pancreatic cancer.

4. The pharmaceutical composition for use according to any one of claims 1 to 3, wherein the pharmaceutical composition is administered intratumorally, intraperitoneally or intravenously.

5. A kit for use in preventing or treating cancer, comprising:
a first composition comprising an oncolytic virus as an active ingredient, wherein the oncolytic virus is a vaccinia virus in which thymidine kinase gene is deleted, and
a second composition comprising hydroxyurea as an active ingredient,
wherein the first composition and the second composition are administered simultaneously, sequentially or in reverse order,
wherein the oncolytic virus is administered at a dose of 1×10⁵ pfu or more pfu,
wherein the hydroxyurea is administered at a dose of 20 mg/kg/day to 90 mg/kg/day,
wherein, when the first composition and the second composition are administered sequentially or in reverse order, the hydroxyurea is continuously administered once a day starting from 3 to 5 days before administration of the oncolytic virus, and continuously administered once a day for 9 to 28 days from 24 hours after administration of the oncolytic virus.

6. The kit for use according to claim 5, wherein the oncolytic virus is administered to the individual at intervals of 7 to 30 days.

7. The kit for use according to claim 5 or claim 6, wherein the first composition and second composition are administered intratumorally, intraperitoneally or intravenously.

## Patentansprüche

1. Pharmazeutische Zusammensetzung zur Verwendung bei der Vorbeugung oder Behandlung von Krebs, die als Wirkstoffe umfasst:
ein onkolytisches Virus, wobei das onkolytische Virus ein Vaccinia-Virus ist, bei dem das Thymidinkinase-Gen deletiert ist,
und Hydroxyharnstoff,
wobei das onkolytische Virus in einer Dosis von 1×10⁵ pfu oder mehr pfu verabreicht wird, und
wobei der Hydroxyharnstoff in einer Dosis von 20 mg/kg/Tag bis 90 mg/kg/Tag verabreicht wird.

2. Pharmazeutische Zusammensetzung zur Verwendung nach Anspruch 1, wobei das onkolytische Virus in einer Dosis von 1×10⁵ pfu bis 1×10¹⁰ pfu verabreicht wird.

3. Pharmazeutische Zusammensetzung zur Verwendung nach Anspruch 1 oder Anspruch 2, wobei der Krebs einer ist, ausgewählt aus der Gruppe bestehend aus Lungenkrebs, Dick- und Enddarmkrebs, Prostatakrebs, Schilddrüsenkrebs, Brustkrebs, Hirnkrebs, Kopf- und Halskrebs, Speiseröhrenkrebs, Hautkrebs, Thymusdruck, Magenkrebs, Dickdarmkrebs, Leberkrebs, Eierstockkrebs, Gebärmutterkrebs, Blasenkrebs, Enddarmkrebs, Gallenblasenkrebs, Gallengangskrebs und Bauchspeicheldrüsenkrebs.

4. Pharmazeutische Zusammensetzung zur Verwendung nach einem der Ansprüche 1 bis 3, wobei die pharmazeutische Zusammensetzung intratumoral, intraperitoneal oder intravenös verabreicht wird.

5. Set zur Verwendung bei der Vorbeugung oder Behandlung von Krebs, umfassend:
eine erste Zusammensetzung, die ein onkolytisches Virus als Wirkstoff umfasst, wobei das onkolytische Virus ein Vaccinia-Virus ist, bei dem das Thymidinkinase-Gen deletiert ist, und
eine zweite Zusammensetzung, die Hydroxyharnstoff als Wirkstoff umfasst,
wobei die erste Zusammensetzung und die zweite Zusammensetzung gleichzeitig, nacheinander oder in umgekehrter Reihenfolge verabreicht werden,
wobei das onkolytische Virus in einer Dosis von 1×10⁵ pfu oder mehr pfu verabreicht wird,
wobei der Hydroxyharnstoff in einer Dosis von 20 mg/kg/Tag bis 90 mg/kg/Tag verabreicht wird,
wobei, wenn die erste Zusammensetzung und die zweite Zusammensetzung nacheinander oder in umgekehrter Reihenfolge verabreicht werden, der Hydroxyharnstoff kontinuierlich einmal täglich verabreicht wird, beginnend ab 3 bis 5 Tage vor Verabreichung des onkolytischen Virus, und kontinuierlich einmal täglich über 9 bis 28 Tage ab 24 Stunden nach Verabreichung des onkolytischen Virus verabreicht wird.

6. Set zur Verwendung nach Anspruch 5, wobei das onkolytische Virus dem Individuum in Intervallen von 7 bis 30 Tagen verabreicht wird.

7. Set zur Verwendung nach Anspruch 5 oder Anspruch 6, wobei die erste Zusammensetzung und die zweite Zusammensetzung intratumoral, intraperitoneal oder intravenös verabreicht werden.

## Revendications

1. Composition pharmaceutique pour une utilisation dans la prévention ou le traitement d'un cancer, comprenant en tant que principes actifs :
un virus oncolytique, dans laquelle le virus oncolytique est un virus de la vaccine dans lequel le gène de la thymidine kinase est supprimé,
et de l'hydroxyurée,
dans laquelle le virus oncolytique est administré à une dose de 1×10⁵ ufp ou plus, et
dans laquelle l'hydroxyurée étant administrée à une dose de 20 mg/kg/jour à 90 mg/kg/jour.

2. Composition pharmaceutique pour une utilisation selon la revendication 1, dans laquelle le virus oncolytique est administré à une dose de 1×10⁵ ufp à 1×10¹⁰ ufp.

3. Composition pharmaceutique pour utilisation selon la revendication 1 ou la revendication 2, dans laquelle le cancer est l'un quelconque sélectionné dans le groupe consistant en un cancer du poumon, un cancer colorectal, un cancer de la prostate, un cancer de la thyroïde, un cancer du sein, un cancer du cerveau, un cancer de la tête et du cou, un cancer de l'oesophage, un cancer de la peau, une pression thymique, un cancer gastrique, un cancer du côlon, un cancer du foie, un cancer des ovaires, un cancer de l'utérus, un cancer de la vessie, un cancer rectal, un cancer de la vésicule biliaire, un cancer des voies biliaires et un cancer du pancréas.

4. Composition pharmaceutique pour une utilisation selon l'une quelconque des revendications 1 à 3, dans laquelle la composition pharmaceutique est administrée dans la tumeur, par voie intrapéritonéale ou par voie intraveineuse.

5. Kit pour une utilisation dans la prévention ou le traitement d'un cancer, comprenant :
une première composition comprenant un virus oncolytique en tant que principe actif, dans lequel le virus oncolytique est un virus de la vaccine dans lequel le gène de la thymidine kinase est supprimé, et
une seconde composition comprenant de l'hydroxyurée en tant que principe actif,
dans lequel la première composition et la seconde composition sont administrées simultanément, séquentiellement ou dans l'ordre inverse,
dans lequel le virus oncolytique est administré à une dose de 1×10⁵ ufp ou plus,
dans lequel l'hydroxyurée étant administrée à une dose de 20 mg/kg/jour à 90 mg/kg/jour,
dans lequel, quand la première composition et la seconde composition sont administrées séquentiellement ou dans l'ordre inverse, l'hydroxyurée est administrée en continu une fois par jour en commençant 3 à 5 jours avant l'administration du virus oncolytique, et administrée en continu une fois par jour pendant 9 à 28 jours 24 heures après l'administration du virus oncolytique.

6. Kit pour une utilisation selon la revendication 5, dans lequel le virus oncolytique est administré à l'individu à des intervalles de 7 à 30 jours.

7. Kit pour une utilisation selon la revendication 5 ou la revendication 6, dans lequel la première composition et la seconde composition sont administrées dans la tumeur, par voie intrapéritonéale ou par voie intraveineuse.
